# EUROPÄISCHE PATENTSCHRIFT

(11) **EP 2 452 607 B1**
(45) Veröffentlichungstag und Bekanntmachung des Hinweises auf die Patenterteilung: **20.08.2014**
(21) Anmeldenummer: 11008985.1
(22) Anmeldetag: 11.11.2011
(51) Int. Cl.: A47L 15/23, A61B 19/00

(54) **Wascharm-Anordnung für einen mehretagigen Waschgut-Aufnahmewagen**
Washing arm assembly for a trolley for holding objects to be cleaned arranged on tiered rack
Agencement de bras de lavage pour un chariot de plusieurs étages pour produits à laver

(30) Priorität: 12.11.2010 DE 102010051200
(43) Veröffentlichungstag der Anmeldung: 16.05.2012
(73) Patentinhaber: Belimed AG, 6300 Zug (CH)
(72) Erfinder: Petric, Samo, 1386 Stari trg pri Lozu (SI); Ammann, Erich, 6274 Eschenbach (CH); Fromberger, Helmut, 84478 Waldkraiburg (DE)
(74) Vertreter: Feldkamp, Rainer

(56) Entgegenhaltungen:
- EP-A1- 1 774 979
- EP-A1- 1 870 113
- EP-A2- 1 743 566
- DE-B3-102005 059 604
- GB-A- 594 601
- US-A- 3 918 644

## Beschreibung

Die Erfindung bezieht sich auf eine Wascharm-Anordnung für einen Waschgut-Aufnahmewagen, der in den Waschraum einer Reinigungsmaschine zum Reinigen von medizinischen, pharmazeutischen und/oder Labor-Utensilien einsetzbar ist, die auf einzelnen übereinander angeordneten Ebenen des Aufnahmewagens anzuordnen sind.

Es sind Waschgut-Aufnahmewagen bekannt, die in den Waschraum einer Reinigungsmaschine beispielsweise nach Art einer Geschirrspülmaschine oder zum Reinigen von medizinischen, pharmazeutischen und/oder Labor-Utensilien einsetzbar sind, wobei der Begriff "Reinigung" hierbei sowohl das Beaufschlagen der einzelnen Utensilien oder Gegenstände mit einer Waschflüssigkeit, einer Sprühflüssigkeit und gegebenenfalls Trocknungsluft einschließen soll. Derartige Waschgut-Aufnahmewagen weisen zumeist mehrere übereinander angeordnete Etagen oder Ebenen auf, in denen das Waschgut entweder direkt oder in Körben angeordnet werden kann, wobei jeder dieser Ebenen zumindest ein Wascharm zugeordnet ist, der um eine vertikale Drehachse drehbar ist, die gleichzeitig zur Zuführung eines Strömungsmediums, wie der Waschflüssigkeit oder Trocknungsluft über Kanäle dient, die beim Einschieben des Waschgut-Aufnahmewagens in den Waschraum mit einer in dem Waschraum angeordneten Zuführungs- und Verteilereinrichtung verbunden werden. Durch entsprechende Gestaltung der Austrittsrichtung der Strömungsmedium-Strahlen aus den Wascharmen wird ein Antrieb der Wascharme um deren Drehachse erreicht. Durch die Verteilereinrichtung und die Zuführung des Strömungsmediums in getrennter Weise an jeden der Wascharme ergeben sich einerseits erhebliche Strömungswiderstände und andererseits Abdichtungs-probleme sowohl zwischen der Verteilereinrichtung in dem Waschraum und dem Aufnahmewagen als auch zwischen den Kanälen in dem Aufnahmewagen und dem sich drehenden Wascharmen.

Aus der EP 1 774 979 A1 ist eine Wascharm-Anordnung bekannt, bei der bei einer Ausführungsform an einer hohlen Drehachse mehrere Drehflügel in verschiedenen Zahlen und Anordnungen axial voneinander beabstandet vorgesehen sind.

Weiterhin ist aus der GB 594,601 eine Wascharm-Anordnung bekannt, bei der ebenfalls ein zentrales Tragrohr eine Anzahl von Wascharmen trägt, die durch eine mit hohem Druck in das obere Ende des Tragrohres eingespeiste Waschflüssigkeit in Drehung versetzt werden.

Aus der EP 1 870 113 A1 ist weiterhin eine Wascharm-Anordnung bekannt, bei der die Wascharme an ihren Enden durch Endkappen verschlossen sind, die ihrerseits an den freien Enden Austrittsdüsen für die Waschflüssigkeit aufweisen.

Der Erfindung liegt die Aufgabe zugrunde, eine Wascharm-Anordnung der eingangs genannten Art zu schaffen, bei der sich eine wesentliche Vereinfachung und vergrößerte Zuverlässigkeit des Drehantriebs der Wascharme ergibt.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Dadurch, dass die einzelnen Wascharme vertikal übereinander an einem zentralen Tragrohr befestigt sind, ist es lediglich erforderlich, an den Enden des Tragrohres abgedichtete Drehlager vorzusehen. Damit werden Abdichtungsprobleme wesentlich verringert, und jeder Wascharm trägt zur Antriebskraft für eine Drehung des gemeinsamen Tragrohres und damit auch der anderen Wascharme bei.

Da eine strömungsmitteldichte Drehlagerung nur an den Enden des Tragrohres und nicht an den einzelnen Wascharmen erforderlich ist, ergibt sich eine wesentliche Vereinfachung des Aufbaus der Wascharm-Anordnung bzw. des Waschgut-Aufnahmewagens und eine verbesserte Antriebskraft-Übertragung auf die Wascharm-Anordnung.

Das Tragrohr wird vorzugsweise lediglich von einem Ende aus mit dem Strömungsmedium gespeist, kann jedoch auch von beiden Enden aus gespeist werden, falls dies gewünscht ist. In jedem Fall ergibt sich eine Vereinfachung der in der Waschkammer angeordneten Zuführungseinrichtungen für das Strömungsmedium an die einzelnen Wascharme, da keine aufwändigen Verteilereinrichtungen und getrennte Drehlager für jeden Wascharm erforderlich sind.

Das Tragrohr weist bei einer Ausführungsform jeweilige Paare von sich diametral gegenüberliegenden radialen Anschlussöffnungen für den Anschluss jeweiliger Hälften eines Wascharms auf. In den Innenraum des Tragrohrs im Bereich der Anschlussöffnungen für die Wascharme sind hierbei vorzugsweise stromabwärts von diesen Anschlussöffnungen liegende Drosselelemente eingesetzt, die eine gleichförmige Druckverteilung über die in einzelnen Höhenebenen angeordneten Wascharme bewirken.

Diese Drosselelemente können an ihren den Anschlussöffnungen für die Wascharme (3) benachbarten Enden Abschrägungen zum Lenken der in dem Tragrohr geführten Waschflüssigkeit in die Wascharme aufweisen.

Gemäß einer bevorzugten Ausgestaltung der Erfindung ist vorgesehen, dass die Drehlager des Tragrohres durch auf das obere und untere Ende des Tragrohres drehfest aufgesetzte Innen-Lagerringe und Außen-Lagerhülsen gebildet sind, in denen die Innen-Lagerringe drehbar gelagert sind und die an unteren und oberen Ebenen des Aufnahmewagens befestigt sind.

Vorzugsweise weisen die Innen-Lagerringe an ihrem Außenumfang eine Labyrinth-Dichtung auf, die eine Abdichtung gegenüber der zugeordneten Außen-Lagerhülse des Aufnahmewagens ergibt.

Zumindest eine der unteren und oberen Lager-Außenhülsen kann einen Teil einer Kupplungeinrichtung zur Kupplung mit einer im Waschraum der Reinigungsmaschine vorgesehenen Kupplungsanordnung für die Zuführung von Waschflüssigkeit bilden.

Die Erfindung wird im Folgenden anhand von der Zeichnung dargestellten Ausführungsbeispielen noch näher erläutert.

In den Zeichnungen zeigen:
Figur 1 eine perspektivische Ansicht einer Ausführungsform eines Waschgut-Aufnahmewagens mit einer Ausführungsform der Wascharm-Anordnung;
Figur 2 eine schematische Vorderansicht des Waschgut-Aufnahmewagens nach Figur 1;
Figur 3 eine Schnittansicht entlang der Linie A-A nach Figur 2 in Betrachtung der Pfeile;
Figur 4 eine Ansicht der Ausführungsform der Wascharm-Anordnung;
Figur 5 eine Schnittansicht der oberen Lagerung des Tragrohres der Wascharm-Anordnung nach Figur 4;
Figur 6 eine Schnittansicht der unteren Lagerung des Tragrohres der Wascharm-Anordnung nach Figur 4;
Figur 7 eine Schnittansicht einer Ausführungsform des Tragrohres mit einem darin eingesetzten Drosselelement;
Figur 8 eine Detailansicht einer Ausführungsform des freien Endes eines Wascharms

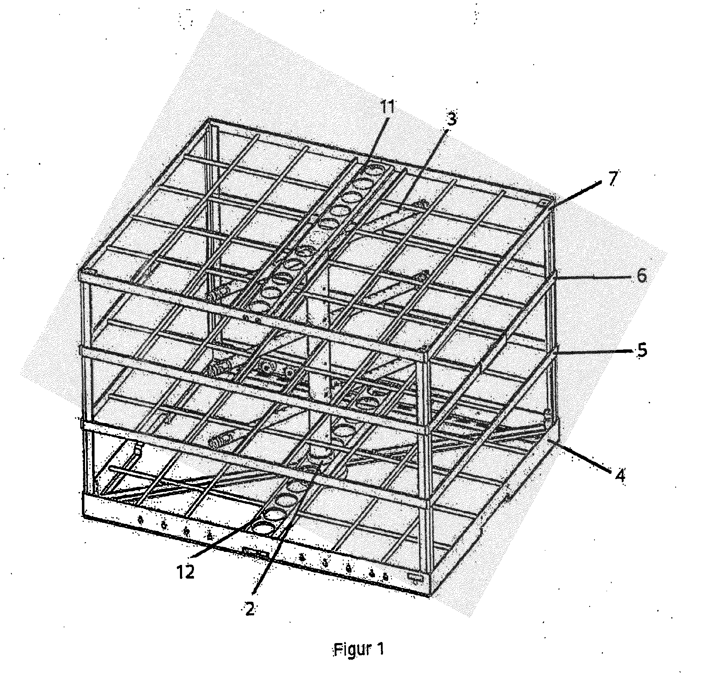

In Figur 1 ist schematisch eine Ansicht eines Waschgut-Aufnahmewagens 1 gezeigt, der in eine nicht gezeigte Aufnahmekammer einer Reinigungs- und/oder Desinfektions- und/oder Trocknungsmaschine (im Folgenden "Reinigungsmaschine") für die Behandlung von medizinischen, pharmazeutischen und/oder Labor-Utensilien einsetzbar oder einschiebbar ist. Dieser Waschgut-Aufnahmewagen 1 weist mehrere Etagen oder Ebenen 4, 5, 6, 7 auf, die übereinander angeordnet sind und mit entsprechenden Auflageflächen für Aufnahmekörbe für zu reinigende Gegenstände oder für die zu reinigenden Gegenstände selbst versehen sind.

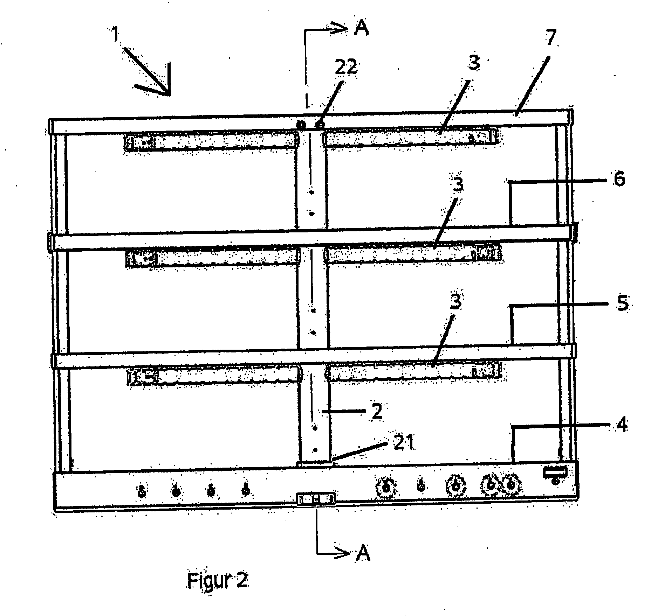

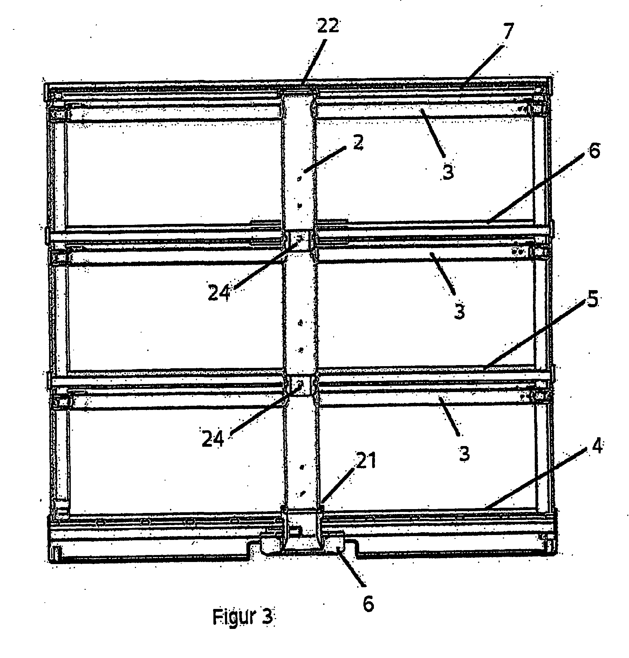

Im Inneren dieses Waschgut-Aufnahmewagens 1 ist eine Anzahl von Wascharmen 3 drehbar angeordnet, die an einem zentralen Tragrohr 2 befestigt sind, das an seinem oberen und unteren Ende an oberen und unteren Ebenen des Aufnahmewagens 1 drehbar befestigt ist, wie dies insbesondere aus den Figuren 2 und 3 zu erkennen ist. Das Tragrohr 2 ist bei der dargestellten Ausführungsform an seinem oberen Ende verschlossen, während sein unteres Ende offen ist und die Zuführung sowohl von Wasch- oder Spülflüssigkeit als auch von Trocknungsluft ermöglicht. An diesem Tragrohr 2 sind die einzelnen Wascharme 3 vorzugsweise jeweils symmetrisch zueinander befestigt, wobei die Wascharme 3 hohl mit Innenräumen ausgebildet sind und mit ihren dem Tragrohr zugewandten Enden über jeweilige Anschlussöffnungen mit einem Innenraum des Tragrohres 2 in Verbindung stehen, so dass bei der dargestellten Ausführungsform von unten in das Tragrohr 2 eingespeiste Waschflüssigkeit oder Trocknungsluft von dem Tragrohr 2 aus in die Innenräume der Wascharme 3 strömen kann. Die Wascharme 3 sind an ihren Enden verschlossen und weisen entlang ihrer Länge Austrittsöffnungen 31 auf, aus denen die Waschflüssigkeit oder Trocknungsluft austreten kann.

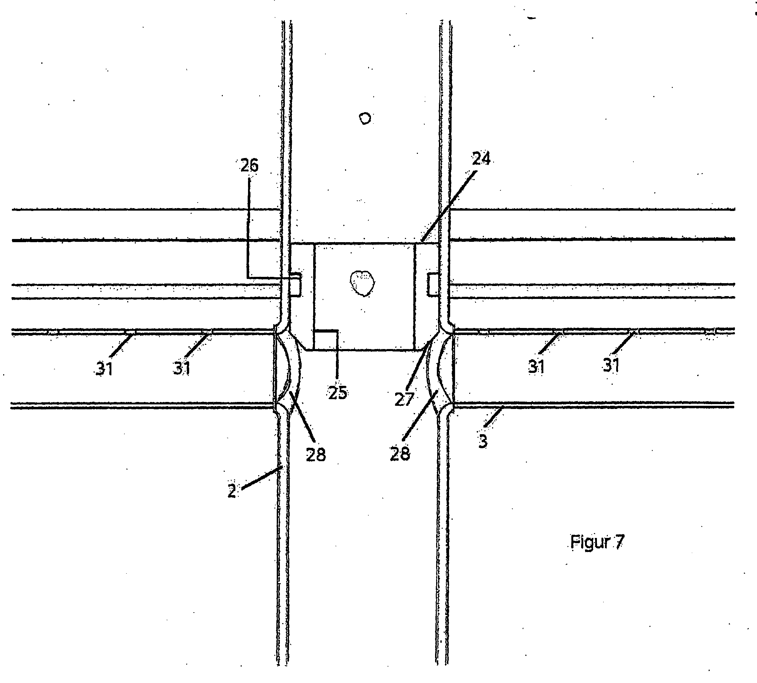

Um trotz der in unterschiedlichen Höhenlagen angeordneten Wascharme 3 eine gleichmäßige Verteilung eines Strömungsmediums, wie der Wasch- oder Spülflüssigkeit oder Trocknungsluft, auf die einzelnen übereinander angeordneten Wascharme 3 zu erreichen, sind in der aus den Figuren 3 und 7 ersichtlichen Weise im Inneren des Tragrohres Drosselelemente 24 angeordnet, die einerseits eine Querschnittsverengung des Querschnittes des Tragrohres 2 und andererseits eine Umlenkung des Strömungsmediums in die einzelnen Tragrohre ermöglichen.

Diese Drosselelemente 24 sind ringförmig ausgebildet und weisen in der insbesondere aus Figur 7 ersichtlichen Weise einen Innenquerschnitt 25 auf, der entsprechend der Position der Drosselelemente 24 in dem Tragrohr eine entsprechende Verteilung der Flüssigkeitsströmung auf die einzelnen Wascharme 3 ermöglicht. An ihren den Anschlussöffnungen 28 des Tragrohres 2 benachbarten Enden weisen die Drosselelemente 24 Abschrägungen 27 auf, die eine Umlenkung des Strömungsmediums in die Innenräume der Wascharme 3 verbessern.

Das Tragrohr 2 kann seinerseits ebenfalls mit Austrittsöffnungen für das Strömungsmedium versehen sein, so dass bei einer Drehung des Tragrohres auch Strahlen des Strömungsmediums auf die in den einzelnen Ebenen angeordneten Gegenstände gerichtet werden.

Die Wascharme 3 sind zusätzlich zu den im Wesentlichen nach oben und nach unten (nicht gezeigt) gerichteten Austrittsöffnungen 31 mit im Wesentlichen horizontal gerichteten Austrittsöffnungen 32, 33 an ihren Enden versehen, wobei der aus diesen Öffnungen austretende Strahl des Strömungsmediums einen Antrieb der aus dem Tragrohr 2 und den Wascharmen 3 bestehenden Wascharm-Anordnung um die Mittelachse des Tragrohres 2 hervorruft.

An den ansonsten verschlossenen freien Enden der Wascharme 3 können weiterhin Austrittsöffnungen 33 angeordnet sein, die eine Reinigung des Waschraums bewirken und/oder zum Antrieb der Wascharm-Anordnung beitragen.

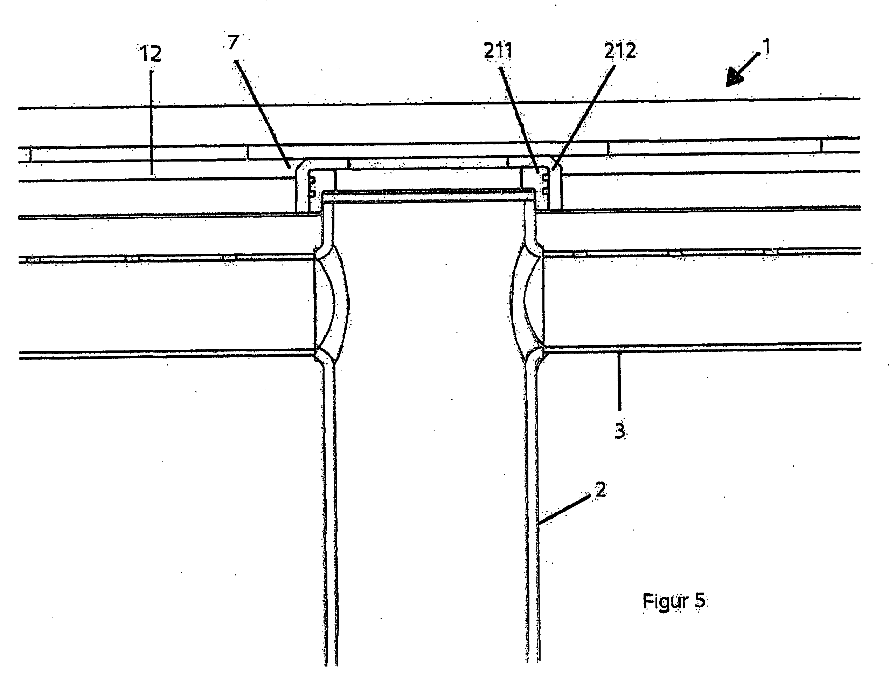

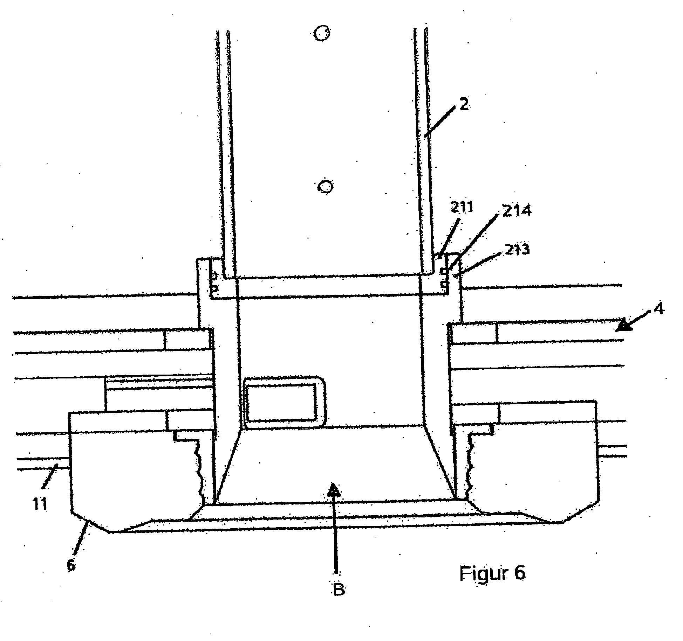

Aus den Figuren 5 und 6 sind Einzelheiten der oberen bzw. unteren Lagerung des Tragrohres 2 zu erkennen. Auf die oberen und unteren Enden des rohrförmigen Tragrohres sind Innen-Lagerringe 211 aufgesetzt, die beispielsweise aus einem gute Gleiteigenschaften aufweisenden Kunststoff bestehen und mit einer Labyrinth-Dichtungs-Außenfläche 214 versehen sind, die in Figur 6 schematisch mit der Bezugsziffer 212 bezeichnet ist, um eine Abdichtung der Enden des Tragrohres 2 gegenüber Außen-Lagerhülsen 212, 213 zu bewirken, die an oberen bzw. unteren Querstreben 11. 12 des Aufnahmewagens 1 befestigt sind.

Wie dies in Figur 6 gezeigt ist, ist das untere Ende des Tragrohres 2 über seinen darauf aufgesetzten Innenlagerring 211 im Inneren einer Außen-Lagerhülse 213 gelagert, die in der unteren Querstrebe 12 des Aufnahmewagens 1 befestigt ist. Diese Außen-Lagerhülse 213 erstreckt sich in ein Kupplungselement 6, das mit einem nicht dargestellten, hiermit zusammenwirkenden Kupplungselement im Waschraum der Reinigungsmaschine, wobei eine strömungsmitteldichte Kupplung beim Einschieben des Aufnahmewagens 1 in die Waschkammer erreicht wird.

Auf diese Weise kann ein Strömungsmedium von der Waschkammer der Maschine aus in das untere Ende des Tragrohres 2 eingeleitet und über dieses Tragrohr 2 auf die einzelnen Wascharme 3 verteilt werden. Anstelle oder zusätzlich zu der Einleitung des Strömungsmediums von unten in das Tragrohr 2 kann auch eine Einleitung des Strömungsmediums von oben in das Tragrohr erfolgen.

Durch die gemeinsame Anordnung und Speisung der jeweiligen Wascharme 2 in den einzelnen Etagen oder Ebenen des Aufnahmewagens 1 mit Hilfe eines gemeinsamen Tragrohres 2 wird eine wesentliche Vereinfachung der Konstruktion des Aufnahmewagens 1 erreicht, da nicht an jeder einzelnen Etage oder Ebene getrennte Lagerungen für Wascharme sowie Zuführeinrichtungen für das Strömungsmedium an diese Wascharme vorgesehen werden müssen.

Da lediglich eine obere und untere Lagerung des Tragrohres vorgesehen sein muss, ergibt sich weiterhin eine geringere Reibung der gesamten Wascharm-Anordnung, so dass sich diese Wascharme leichter bei einem vorgegebenen Strömungsmitteldruck drehen können. Weiterhin tragen die im Wesentlichen horizontal ausgerichteten Austrittsöffnungen 32 sämtlicher Wascharme zum Antrieb der Wascharm-Anordnung bei.

Sowohl das Tragrohr 2 als auch die einzelnen Wascharme 3 können aus Rohrmaterial mit kreisförmigem Querschnitt hergestellt sein, und die Wascharm-Hälften können mit dem Tragrohr verschweißt und an ihren freien Enden durch Verschlusskappen verschlossen werden, so dass die Fertigung der Wascharm-Anordnung vereinfacht wird.

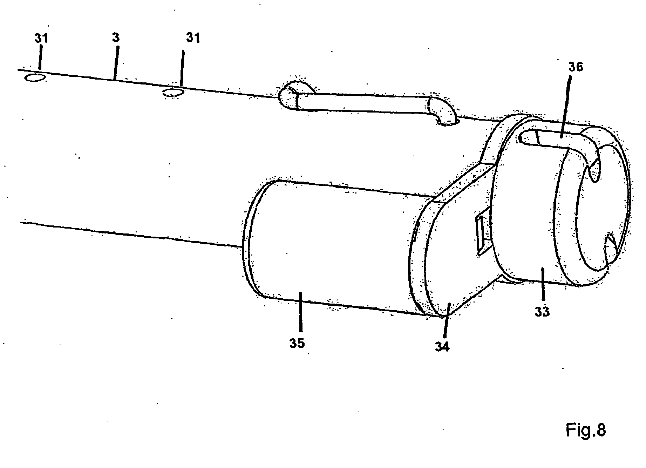

Wie dies in Figur 8 gezeigt ist, können die freien Enden der Wascharme 3 durch Endkappen 33 verschlossen sein, in denen weitere Austrittsöffnungen oder Düsen 36 derart angeordnet sind, dass in Längs- und Vertikalrichtung der Wascharme 3 gerichtete, beispielsweise fächerförmige Waschflüssigkeitsstrahlen erzeugt werden.

An den Endkappen 33 zumindest eines der Wascharme können weiterhin Halter 34 für zumindest einen Magneten 35 derart angeordnet sein, dass der zumindest eine Magnet 35 gegenüber der Längsachse des Wascharms achsversetzt angeordnet ist, wobei die Magnete mit ortsfest angeordneten Magnet-Sensoren für eine Überwachung der Drehung des Wascharms 3 zusammenwirken.

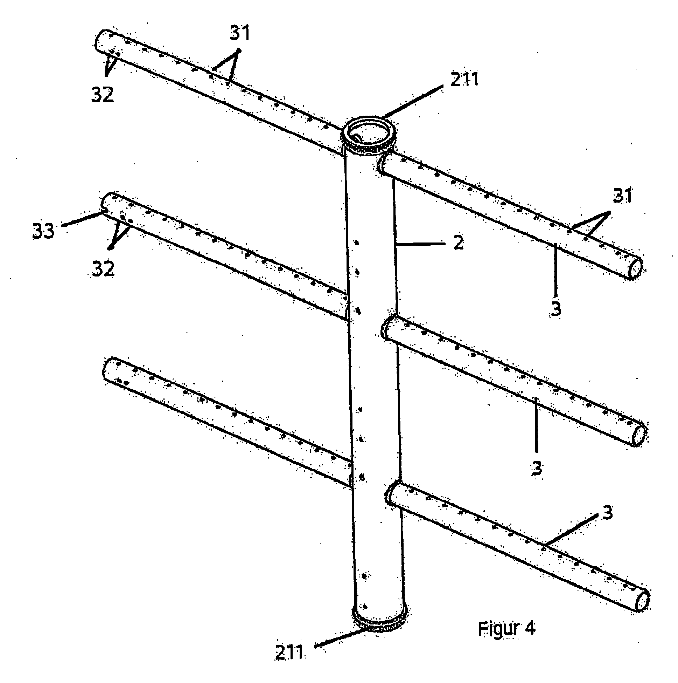

Obwohl bei der Ausführungsform der Wascharme, wie sie insbesondere aus Figur 4 zu erkennen ist, die Austrittsöffnungen 31 in einer vertikalen Ausrichtung entlang einer Linie über die Länge des Wascharms ausgerichtet sind, können diese Öffnungen in den einzelnen Wascharm-Hälften auch entlang einer Linie ausgerichtet sein, die gegenüber einem vertikalen Durchmesser des Wascharms 3 unter einem derartigen Winkel angeordnet ist, dass auch die Austrittsöffnungen 31 zur Ausübung einer Drehkraft auf die durch die Wascharme 3 und das Tragrohr 2 gebildete Wascharm-Anordnung beitragen.

Bei einer anderen Ausführungsform können die Öffnungen 31 auf beiden Hälften des Wascharms bei Betrachtung von einem Ende eines Wascharms 3 aus entlang einer Linie angeordnet sein, die in beiden Hälften gegenüber einem vertikalen Durchmesser des Wascharms 3 unter dem gleichen vorgegebenen Winkel versetzt ist, so dass sich eine verschränkte Anordnung der Strahlen bei einer Drehung des Wascharms um 180 Grad ergibt, um die Waschwirkung auf die Gegenstände zu verbessern, die auf den einzelnen Etagen oder Ebenen angeordnet sind.

Letzteres ist aufgrund der zusätzlichen Austrittsöffnungen 32, 33 möglich, die den Hauptantrieb der Wascharme 3 um die Mittelachse des Tragrohres 2 bewirken.

## Patentansprüche

1. Wascharm-Anordnung für einen Waschgut-Aufnahmewagens (1), der in den Waschraum einer Reinigungsmaschine zum Reinigen von medizinischen, pharmazeutischen und/oder Labor-Utensilien einsetzbar ist, die auf einzelnen übereinander angeordneten Ebenen (4, 5, 6, 7) des Aufnahmewagens (1) anzuordnen sind, wobei die Wascharm-Anordnung den jeweiligen Ebenen (4, 5, 6, 7) zugeordnete, vertikal übereinander an einem zentralen Tragrohr (2) befestigte Wascharme (3) aufweist, die um eine vertikale Drehachse drehbar sind und jeweils Innenräume aufweisen, die mit dem Innenraum des Tragrohres (2) in Strömungsmittel-Verbindung stehen und von dem Tragrohr (2) aus mit Waschflüssigkeit gespeist werden, und die mit Austrittsöffnungen (31, 32, 33) versehen sind, aus denen Waschflüssigkeits-Strahlen auf die auf den einzelnen Ebenen (4, 5 6, 7) des Aufnahmewagens (1) befindlichen Gegenstände lenkbar sind, wobei das Tragrohr so ausgebildet ist, dass es an seinen Enden in Drehlagern (21, 22) in den oberen und unteren Ebenen (4, 7) des Aufnahmewagens drehbar lagerbar ist und die Waschflüssigkeit in einen Innenraum des Tragrohrs (2) an zumindest einem Ende hiervon einspeisbar ist, **dadurch gekennzeichnet,**
**dass** das Tragrohr an seinen Enden in Drehlagern (21, 22) in den unteren und oberen Ebenen (4, 7) des Aufnahmewagens (1) drehbar gelagert ist,
**dass** das Tragrohr (2) jeweilige Paare von sich diametral gegenüberliegenden radialen Anschlussöffnungen für den Anschluss jeweiliger Hälften eines Wascharms (3) aufweist,
**dass** in den Innenraum des Tragrohrs (2) im Bereich der Anschlussöffnungen für die Wascharme Drosselelemente (24) eingesetzt sind, die eine gleichförmige Druckverteilung über die in einzelnen Höhenebenen angeordneten Wascharme (3) bewirken.

2. Wascharm-Anordnung nach Anspruch 1, **dadurch gekennzeichnet, dass** die Drosselelemente (24) an ihren den Anschlussöffnungen für die Wascharme (3) benachbarten Enden Abschrägungen (27) zum Lenken der in dem Tragrohr (2) geführten Waschflüssigkeit in die Wascharme (3) aufweisen.

3. Wascharm-Anordnung nach Anspruch 1 oder 2, **dadurch gekennzeichnet, dass** der Außenumfang der Drosselelemente (24) gegenüber der Innenwand des Tragrohres (2) abgedichtet ist und dass die Drosselelemente (24) eine zentrale Durchgangsöffnung (25) aufweisen.

4. Wascharm-Anordnung nach einem der Ansprüch 1 bis 3, **dadurch gekennzeichnet, dass** die Drehlager (21, 22) durch auf das obere und untere Ende des Tragrohres (2) drehfest aufgesetzte Innen-Lagerringe (211) und Außen-Lagerhülsen (213, 212) gebildet sind, in denen die Innen-Lagerringe (211) drehbar gelagert sind und die an unteren und oberen Ebenen (4, 7) des Aufnahmewagens befestigt sind, und dass zumindest eine der unteren und oberen Drehlager (21, 22) einen Teil einer Kupplungeinrichtung (6) zur Kupplung mit einer im Waschraum der Reinigungsmaschine vorgesehenen Kupplungsanordnung für die Zuführung von Waschflüssigkeit an das Tragrohr (2) bildet.

5. Wascharm-Anordnung nach Anspruch 4, **dadurch gekennzeichnet, dass** die Innen-Lagerringe (211) an ihrem Außenumfang eine Labyrinth-Dichtung (214) bilden, die eine Abdichtung gegenüber der zugeordneten Außen-Lagerhülse (213, 212) des Aufnahmewagens (1) bildet.

## Claims

1. Washing arm assembly for a trolley (1) for holding objects to be cleaned, said trolley being adapted to be introduced into a washing compartment of a cleaning machine for cleaning medical, pharmaceutical and/or laboratory utensils, which are arranged on individual racks (4, 5, 6, 7) disposed one above the other of the trolley (1), the washing arm assembly comprising washing arms (3) associated with the respective racks (4, 5, 6, 7) and secured one vertically above the other to a central support tube (2), said washing arms (3) being rotatable about a vertical axis of rotation and each have interior spaces being in fluid connection with the interior space of the support tube (2) and which are fed with washing fluid from the support tube (2), the washing arms being provided with exit openings (31, 32, 33) from which washing fluid jets may be directed onto objects disposed on the respective racks (4, 5, 6, 7) of the trolley (4), the support tube being adapted for being supported at its ends in rotary bearings (21, 22) on the lowermost and uppermost racks of the trolley and for washing fluid to be introduced into an interior space of the support tube (2) through at least one end thereof, **characterized in that**:
the support tube (2) comprises respective pairs of diametrically opposed radial connection openings for the attachment of respective halves of a washing arm (3),
throttle elements (24) being installed into the interior space of the support tube (2) in the area of the connection openings for the washing arms, said throttle elements being adapted to ensure a uniform pressure distribution over the washing arms (3) disposed at the individual levels.

2. Washing arm assembly according to claim 1, **characterized in that** the throttle elements (24) comprise, at their ends adjacent to the connection openings for the washing arms (3), bevels (27) for directing the washing fluid conducted through the support tube (2) into the washing arms (3).

3. Washing arm assembly according to claim 1 or 2, **characterized in that** the outer surface of the throttle elements (24) is sealed off against the inside wall of the support post (2), and **in that** the throttle elements (24) comprise a central through opening (25).

4. Washing arm assembly according to any of the claims 1 through 3, **characterized in that** the rotary bearings (21, 22) are formed by inner bearing rings mounted in a torque-proof manner on the upper and lower ends of the support tube, and by outer bearing bushes (213, 212) within in which the inner bearing rings (211) are rotatably supported, and which are fastened to the lowermost and uppermost racks (4, 7) of the trolley, and **in that** at least one of the lower and upper rotary bearings (21, 22) forms part of a coupling device (6) for coupling to a coupling arrangement provided in the washing compartment for supplying washing fluid to the support tube (2).

5. Washing arm assembly according to claim 4, **characterized in that** the inner bearing rings (211) form on their outer circumference a labyrinth seal (214) which forms a seal against the associated outer bearing bush (213, 212) of the trolley (1).

## Revendications

1. Agencement de bras de lavage pour un chariot de réception (1) de produits à laver qui peut être utilisé dans le compartiment de lavage d'une machine de nettoyage pour nettoyer des ustensiles médicaux, pharmaceutiques et/ou de laboratoire, qui sont à disposer sur plusieurs plans (4, 5, 6, 7) individuels disposés les uns sur les autres du chariot de réception (1), sachant que l'agencement de bras de lavage présente des bras de lavage (3) correspondant aux plans (4, 5, 6, 7) respectifs, fixés verticalement les uns au-dessus des autres sur un tube porteur (2) central, qui peuvent tourner sur un axe de rotation vertical et présentent respectivement des espaces intérieurs qui sont reliés en écoulement avec l'espace intérieur du tube porteur (2) et sont alimentés en liquide de lavage par le tube porteur (2), et qui sont dotés d'ouvertures d'évacuation (31, 32, 33), desquelles des jets de liquide de lavage peuvent être dirigés sur les objets se trouvant sur les différents plans (4, 5, 6, 7) du chariot de réception (1), sachant que le tube porteur est ainsi formé qu'il peut être supporté en rotation dans les plans inférieurs et supérieurs du système de réception de produits à laver par ses extrémités dans des paliers rotatifs (21, 22), et le liquide de lavage peut être alimenté dans un espace intérieur du tube porteur (2) sur au moins une extrémité de celui-ci, **caractérisé en ce que**
le tube porteur (2) présente des paires respectives d'ouvertures de raccordement radiales diamétralement opposées pour raccorder les moitiés respectives d'un bras de lavage (3),
que des éléments d'étranglement (24) sont introduits dans l'espace intérieur du tube porteur (2) au niveau des ouvertures de raccordement pour les bras de lavage, lesquels sont ainsi formés qu'ils effectuent une répartition égale de la pression sur les bras de lavage (3) disposés à différentes hauteurs.

2. Bras de lavage selon la revendication 1, **caractérisé en ce que** les éléments d'étranglement (24) présentent des chanfreins (27) sur leurs extrémités voisines des ouvertures de raccordement pour les bras de lavage (3), pour diriger dans les bras de lavage (3) le liquide de lavage dirigé dans le tube porteur (2).

3. Bras de lavage selon la revendication 1 ou 2, **caractérisé en ce que** le pourtour extérieur des éléments d'étranglement (24) est étanchéifié par rapport à la paroi intérieure du tube porteur (2) et que les éléments d'étranglement (24) présentent une ouverture de passage centrale (25).

4. Bras de lavage selon l'une des revendications 1 à 3, **caractérisé en ce que** les paliers rotatifs (21, 22) sont formés par des bagues de palier intérieures (211) et des douilles de palier extérieures (213, 212) disposées fixes en rotation sur les extrémités supérieure et inférieure du tube porteur (2), dans lesquelles les bagues de palier intérieures (211) sont disposées de manière à pouvoir tourner et sont fixées sur les plans supérieur et inférieur (4, 7) du chariot de réception, et qu'au moins un des paliers rotatifs (21, 22) inférieur et supérieur forme une partie d'un dispositif de couplage (6) pour le couplage à un agencement de couplage prévu dans l'espace de lavage de la machine de nettoyage pour amener le liquide de lavage au tube porteur (2).

5. Bras de lavage selon la revendication 4, **caractérisé en ce que** les bagues de palier intérieures (211) forment un joint labyrinthe (214) sur leur pourtour extérieur, qui forme une étanchéité par rapport à la douille de palier extérieure (213, 212) correspondante du chariot de réception (1).
